# EUROPEAN PATENT APPLICATION

(11) **EP 4 527 370 A1**
(43) Date of publication of application: **26.03.2025**
(21) Application number: 24199263.5
(22) Date of filing: 09.09.2024
(51) Int. Cl.: A61K 8/34, A61K 8/42, A61K 8/9789, A61P 17/02, A61Q 19/00

(54) **DERMATOLOGICAL AND COSMETIC COMPOSITION, IN PARTICULAR FOR TREATING HYPERPIGMENTATION AND OF RELATED SKIN IRRITATIONS**

(30) Priority: 15.09.2023 IT 202300019020
(71) Applicant: FB Dermo srl, 20149 Milano (IT)
(72) Inventor: BANFI, Fabio, Milano (IT)
(74) Representative: Manfredi, Irene

(57) **Abstract**

The present invention relates to a composition able to offer significant relief in the treatment of skin hyperpigmentation and of skin inflammation and/or irritation. Furthermore, the present invention also relates to pharmaceutical products comprising said composition of the invention with suitable pharmaceutically acceptable excipients, and to the use of the same in the treatment of skin hyperpigmentation and/or dyschromia, and for use in the treatment of skin inflammatory diseases and/or irritant disturbances, optionally associated to hyperpigmentation and/or dyschromia.

## Description

### FIELD OF THE INVENTION

The present invention relates to a dermatological and/or cosmetic composition able to offer significant improvements in the treatment of skin hyperpigmentation and/or dyschromia, and in the treatment of skin inflammation and/or irritation. Furthermore, the present invention also relates to pharmaceutical products comprising the composition of the invention and suitable pharmaceutically acceptable excipients, and to the use of the same in the treatment of skin hyperpigmentation and/or dyschromia, and of skin inflammatory diseases and/or irritant disturbances, optionally associated to hyperpigmentation and/or dyschromia.

### BACKGROUND ART

Melanin is a pigment, physiologically produced by melanocytes as defense from UV radiations.

Uneven skin pigmentation (hyperpigmentation or as it is often known) is a common skin complaint.

Hyperpigmentation is caused by an increase in melanin, appearing as darkened patches or spots on the skin. A number of factors can trigger an increase in melanin production, but the main ones are sun exposure, hormonal influences, age and skin injuries or inflammation.

Spots caused by sun exposure are in fact known as "sun spots" or as "age spots". They appear mainly on body parts that are frequently exposed such as the face, neck, décolleté, hands and arms. They tend to be small, darkened patches of skin.

Once dark spots have developed, sun exposure can also exacerbate the issue by making spots even darker.

Hormonal influences are the main cause of a particular kind of hyperpigmentation known as melasma or chloasma. It is thought to occur when the female sex hormones oestrogen and progesterone stimulate the overproduction of melanin when skin is exposed to the sun. In melasma larger patches of hyperpigmentation develop mainly on the face.

Although it can affect both men and women, melasma is most common in women. Melasma occurs in 10-15 percent of pregnant women and in 10-25 percent of women taking oral contraceptives 1 and is sometimes referred to as "the mask of pregnancy". Hyperpigmentation is also symptomatic of certain illnesses such as some autoimmune and gastrointestinal diseases, metabolic disorders and vitamin deficiencies.

Other factors that can cause patches of skin to become darker also include scarring, birthmarks, solar or actinic keratoses and skin cancers.

Furthermore, hyperpigmentation can be triggered by certain medications such as chemotherapy drugs, antibiotics, antimalarials anti-seizure drugs and certain hormone treatments.

It can also be caused by cosmetic procedures such as dermabrasion, laser treatment and chemical peels. Skin is left darkened and discoloured after the wound has healed. Hyperpigmentation, and/or dyschromia, is often concomitant (or a consequence of) skin inflammation and irritation.

Hyperpigmentation and/or dyschromia can in fact also occurs as following skin injury or inflammation such as: cuts, burns, chemical exposure, acne, Atopic Dermatitis or Psoriasis. In these cases, it is called "post-inflammatory" hyperpigmentation or dyschromia and it is typically accompanied by redness, swelling, oozing, scaling and itching.

In inflammatory and/or irritant disturbances of the skin, the stratum corneum, composed of the differentiated keratinocytes, called corneocytes, is often compromised and interleukins (IL), are released.

Inflammation is in fact the consequence of the production of molecules capable of triggering oxidative mechanisms that in turn trigger the production of proinflammatory cytokines by keratinocytes and release of histamine by mast cells.

Dermatological treatments such as chemical peels and laser therapy can help to reduce hyperpigmentation, still the treatments themselves can cause irritation and inflammations. Topical agents also exist, such as hydroquinone, which however can only be used for limited periods of time because, like other forms of chemical peel and laser treatment, it can irritate skin and actually cause post-inflammatory hyperpigmentation, especially in people with phototype 3 - 6 (according to Fitzpatrick phototyping scale).

Currently, steroids, topical immunosuppressants, topical antibiotics, and antihistamines are used as treatments for inflammatory skin disturbances. However, drugs for the treatment of skin disorders can themselves cause skin reactions, thus worsening the skin condition instead of ameliorating it. In particular, if used for a long time, treatments for inflammatory skin diseases can cause swelling striae, skin atrophy, capillary dilation, steroid acne, hirsutism, purpura, and other side effects.

Dermo-cosmetic solutions, that are non-invasive and can be used for long periods, are thus needed to reduce hyperpigmentation, dyschromia, and /or inflammation.

The object of the present invention is therefore the provision of an effective composition for treating hyperpigmentation, dyschromia, and/or inflammatory skin diseases and/or irritant disturbances, which is also well tolerated by the body and which can also be used for long periods, while minimizing side effects.

### SUMMARY OF THE INVENTION

Said object has been achieved by the dermatological and/or cosmetic composition of the present invention, as reported in the claims.

In another aspect, the present invention relates to a pharmaceutical product comprising said composition and suitable pharmaceutically acceptable excipients.

In further aspects, the present invention relates to said pharmaceutical product, further comprising at least one further dermatological active agent, preferably selected from further anti-inflammatory agents, cortisonic agents, antibiotics, antihistaminic agents, anti-acne agents, anti-dandruff agents, anti-psoriasis agents, antifungal agents, antibacterial agents, anti-seborrheic agents, keratolytic agents, humectants, anti-free radical agents, antioxidants, vitamins, and mixtures thereof.

Furthermore, the present invention relates to the composition, or the pharmaceutical product comprising the same, for use in the treatment of skin hyperpigmentation and/or dyschromia, and/or of skin inflammatory diseases and/or irritant disturbances, which can optionally be associated to hyperpigmentation or dyschromia.

In accordance with the present disclosure, a composition or product is "cosmetic" when it provides a cosmetic, non-therapeutic effect; a composition or product is instead "dermatological", when it provides a therapeutic effect.

### BRIEF DESCRIPTION OF THE FIGURES

The characteristics and the advantages of the present invention will become clear from the following detailed description, the working examples provided for illustrative purposes and the accompanying figure, wherein:

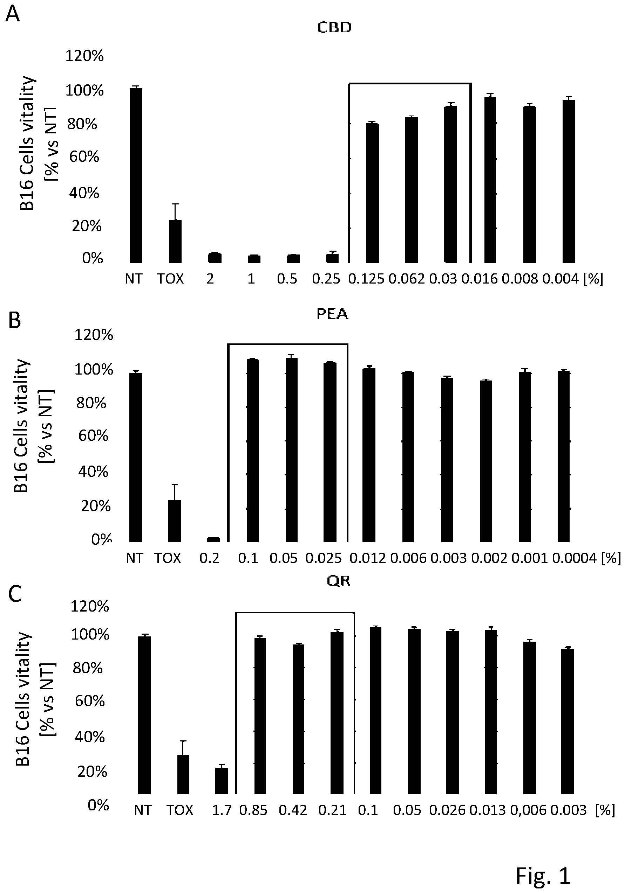

- Fig. 1 shows the results of cytotoxicity tests on B16 cells treated for 24 hours at various concentrations of Cannabidiol (CBD, panel A), Palmitoylethanolamide (PEA, panel B) and Quercus Robur Root Extract (QR, panel C). Values are expressed as 595 nm absorbance percentage over non-treated (NT) cells; 1 mg/ml SDS is used as positive control (TOX); the boxes show the three highest non-toxic concentrations of each compound.

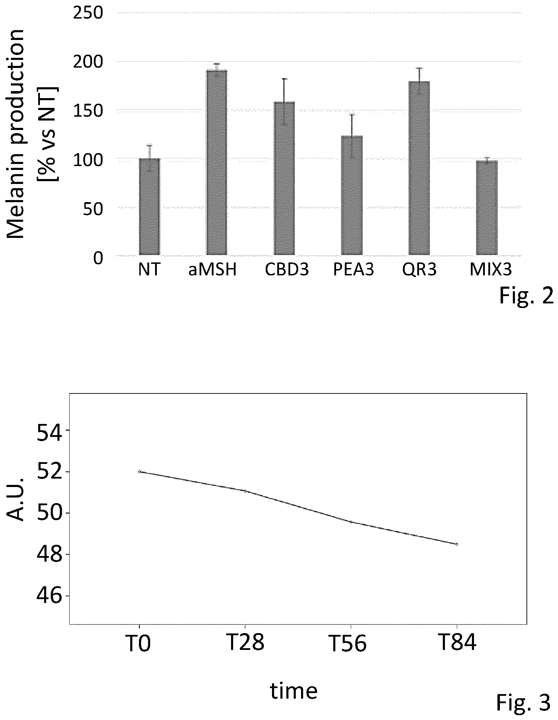

- Fig. 2 shows the results of melanin dosage assay, after induction with aMSH (positive control) and treatment with Cannabidiol (CBD), Palmitoylethanolamide (PEA) and Quercus Robur Root Extract (QR) alone, or with the three in admixture. Values are expressed as percentage of melanin production over non-treated (NT) cells.
- Fig. 3 shows the trend of the variable dark spots' pigmentation at T0 and at the follow-up time points T28, T56 and T84 in the clinical trial of Example 4. The results are expressed as arbitrary units (A.U.)

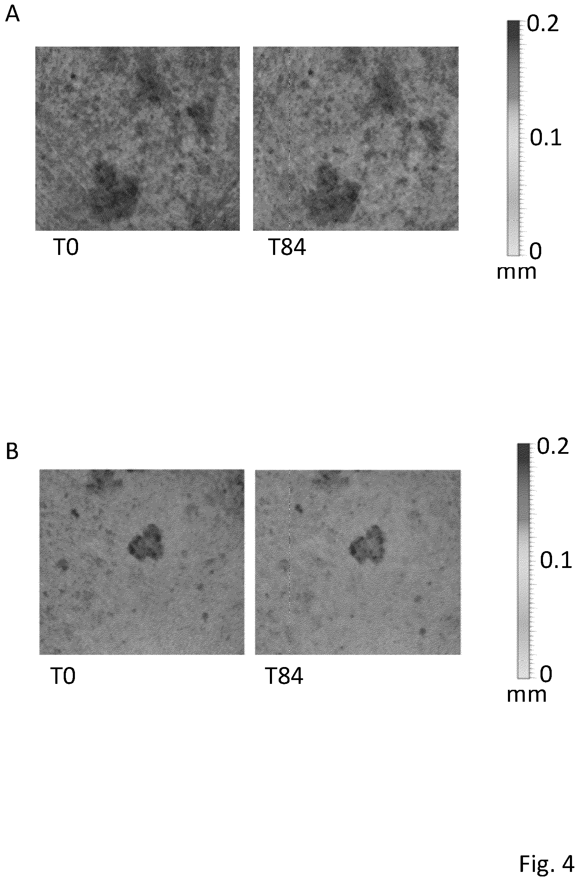

- Fig. 4 shows representative images of spots at T0 and at T84 after treatment with the composition of the invention. Images are detected and analysed with MIRAVEX ANTERA 3D^{®}.

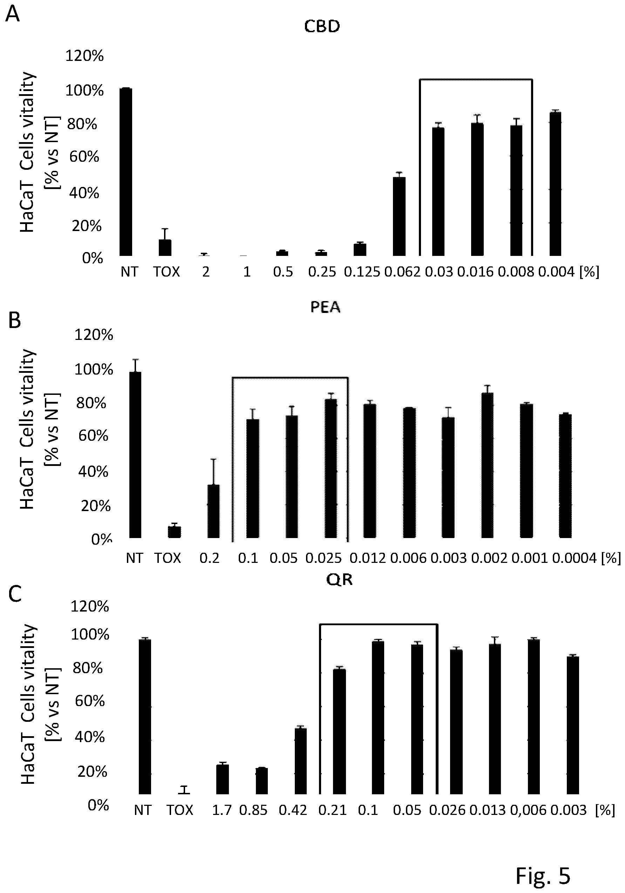

- Fig. 5 shows the results of cytotoxicity tests on HaCaT cells treated for 24 hours at various concentrations of Cannabidiol (CBD, panel A), Palmitoylethanolamide (PEA, panel B) and Quercus Robur Root Extract (QR, panel C). Values are expressed as 595 nm absorbance percentage over untreated (NT) cells; 1 mg/ml SDS is used as positive control; the boxes show the three highest non-toxic concentrations of each compound.

### DETAILED DESCRIPTION OF THE INVENTION

The composition of the invention comprises:
a) oak roots extract;
b) palmitoylethanolamide, or a derivative thereof; and
c) at least one cannabinoid.

Plant meristem cells are rich in substances physiologically active to support and protect cell growth. They are especially rich in phenylpropanoids that play a very important role in defending the plant against external stresses.

The oak roots extract (*Quercus robur* L. roots extract, hereinafter also called QR extract or QR, for brevity) is a botanical extract that is preferably obtainable by water extraction from meristem of oak roots. Such extract is identified by CAS number 71011-28-4.

Palmitoylethanolamide (PEA) belongs to a group of endogenous molecules known as acylethanolamides. PEA is in particular an N-(long-chain-acyl) ethanolamine, that is the ethanolamide of palmitic (hexadecenoic) acid. Derivatives of PEA include, among others, polyethylene glycol derivatives and oxazoline derivatives (such as adelmidrol). Cannabinoids according to the present invention can be of natural or, preferably, of synthetic origin. Synthetic cannabinoids can be produced semi-synthetically, i.e. from natural cannabinoids, or fully synthetically, i.e. from simple basic substances, such as for example limonene.

Preferably, the at least one cannabinoid is cannabidiol (CBD), cannabidiolic acid (CBDA), cannabinol (CBN), cannabigerol (CBG), cannabichromene (CBC), cannabicyclol (CBL), cannabivarin (CBV), tetrahydrocannabivarin (THCV), cannabidivarin (CBDV), cannabichromevarin (CBCV), cannabigervarin (CBGV), cannabigerol monomethyl ether (CBGM), cannabielsoin (CBE), cannabicitran (CBT), or any mixture thereof.

Preferably, the at least one cannabinoid is cannabigerol (CBG) or cannabidiol (CBD), or a mixture thereof. More preferably, the at least one cannabinoid is cannabidiol (CBD).

According to preferred embodiments, the composition of the invention comprises:
a) oak roots extract;
b) palmitoylethanolamide, or a derivative thereof; and
c) at least one cannabinoid being CBD.

According to particularly preferred embodiments, the composition of the invention comprises:
a) oak roots extract;
b) palmitoylethanolamide; and
c) CBD.

Preferably, in the composition of the present invention, the palmitoylethanolamide (PEA), or a derivative thereof, and the oak roots extract (QR) are in a weight ratio of from 1:1 to 1:10, more preferably of from 1:1 to 1:5, most preferably of from 1:1 to 1:3.

Preferably, in the composition of the present invention, the palmitoylethanolamide (PEA), or a derivative thereof, and the at least one cannabinoid are in a weight ratio of from 10:1 to 1:10, more preferably of from 5:1 to 1:10, most preferably of from 1:1 to 1: 5.

Preferably, in the composition of the present invention, the at least one cannabinoid and the oak roots extract (QR) are in a weight ratio of from 10:1 to 1:10, more preferably of from 5:1 to 1:5, most preferably of from 2:1 to 1:2.

According to preferred embodiments, the composition of the present invention comprises a concentration by weight of PEA, or a derivative thereof, of from 0.01 to 0.2 %, more preferably of from 0.02 to 0.1%, most preferably of from 0.02 to 0.05%, based on the total weight of the composition.

According to further, or alternative, embodiments, the composition of the present invention comprises a concentration by weight of the at least one cannabinoid of from 0.01 to 1%, more preferably from 0.02 to 0.5%, most preferably of from 0.05 to 0.1%, based on the total weight of the composition.

According to further, or alternative, embodiments, the composition of the present invention comprises a concentration by weight of the oak roots extract of from 0.01 to 1%, more preferably from 0.05 to 0.5%, most preferably of from 0.1 to 0.2%, based on the total weight of the composition.

The total weight of the composition is preferably the sum of the weight of ingredients a), b) and c) and the weight of suitable vehicle(s) present in the composition. Suitable vehicle(s) can be for instance water, surfactants, or mixtures thereof.

In accordance with the present invention the terms "wt %" or "weight concentration", when referring to the percentage of a component in a composition or product, mean the percentage of the weight of the component, while the terms "v %" or "volume concentration", when referring to the percentage of a component in a composition or product, mean the percentage of the volume of the component, either relative to the total weight of the composition or product or to the total volume of the composition or product, as specified. For instance, the terms "% w/v" mean the percentage of the weight of the component to the total volume of the composition or product. The terms "w/w" or "concentration by weight", when referring to the percentage of a component in a composition or product, means the percentage of the weight of the component, relative to the total weight of the composition or product. The terms "v/v" or "concentration by volume", when referring to the percentage of a component in a composition or product, means the percentage of the volume of the component, relative to the total volume of the composition or product.

Preferably, the composition of the invention is a liquid or semi-liquid solution, comprising:
a) 0.01-0.5% (w/v) of oak roots extract;
b) 0.01-0.1% (w/v) of palmitoylethanolamide, or a derivative thereof; and
c) 0.01-1% (w/v) of at least one cannabinoid.

More preferably, the composition comprises:
a) 0.05-0.1% (w/v) oak roots extract;
b) 0.02-0.05% (w/v) palmitoylethanolamide, or a derivative thereof; and
c) 0.1-0.5% (w/v) of at least one cannabinoid.

The invention further relates to the composition of the invention for use in the treatment of skin hyperpigmentation and/or dyschromia, and/or for use in the treatment of skin inflammatory disease and/or irritant disturbances, optionally associated with hyperpigmentation and/or dyschromia.

The term "treatment" refers to the effects of the composition of the invention, which is capable of providing a benefit to patients suffering from hyperpigmentation and/or skin inflammatory disease and/or irritant disturbances, for example, an improvement in the patient's condition or a delay in the progression of the disease.

Skin inflammatory diseases preferably include ichthyosis, acne, psoriasis, atopic dermatitis (eczema), rosacea, skin redness. Irritant disturbances include skin rashes, urticaria, and itch.

Preferably, the invention further relates to the composition of the invention for use in the treatment of skin hyperpigmentation and/or dyschromia.

The composition of the invention has moreover proved to be effective in reducing inflammation in keratinocytes, as demonstrated in the examples provided hereby, by reduction of inflammation marker IL-6 and IL-8 after TNF-alpha stimulus.

Moreover, the composition of the invention proved to be effective in reducing hyperpigmentation in melanocytes.

In particular, the ingredients of the composition of the invention show an unexpected and significant synergic effect in reducing either inflammation and hyperpigmentation. Preferably, the composition of the invention is to be administered via topical route, more preferably via external topical, sub-cutaneous topical, mucosal topical, gingival topical, intravesical topical, vaginal topical, rectal topical, or ocular topical route.

Preferably, said composition is to be administered in a dose of 0.1-1500 mg per day, the effective dosage being a function of the extent and severity of the skin disease to be treated. In preferred embodiments, said composition is to be administered via external topical route in a dose of 1-1000 mg per day.

In preferred embodiments, the a) oak roots extract, the b) palmitoylethanolamide, or a derivative thereof, and the c) at least one cannabinoid, are the only active ingredients for the treatment of inflammatory skin conditions or diseases, that are present in the composition of the invention.

In further aspects, the present invention relates to a pharmaceutical product comprising the composition of the invention and pharmaceutically acceptable excipients.

The term "excipient" herein means any substance, not itself an active agent, which may be used as a carrier or vehicle for delivery of an active agent to a subject, or combined with an active agent to improve its handling or storage properties, or to permit or facilitate formation of a dose unit of the composition.

Said pharmaceutically acceptable excipients can be rheological additives, buffering agents, antioxidant agents, anti-isothermal agents, antistatic agents, absorbent agents, UV absorbing agents, astringent agents, chelating agents, skin conditioning agents, preservative agents, covering agents, denaturing agents, depigmenting agents, emulsifying agents, film-forming agents, gelling agents, moisturizing agents, hydrotropic agents, binders, soothing agents, smoothing agents, opacifying agents, plasticizing agents, propelling agents, skin protecting agents, reducing agents, cooling agents, sebum-restoring agents, solvents , stabilizing agents, emulsifying stabilizing agents, toning agents, wetting agents, volumizing agents or combinations thereof.

Preferably, the pharmaceutical product of the invention comprises at least one surfactant, more preferably at 1-15% (w/w).

According to preferred embodiments, the pharmaceutical product of the invention, further comprises one or more of: a humectant, preferably at 1 - 10% (w/w); a thickening agent, preferably at 0.01 - 10% (w/w); a gel-forming agent, preferably at 0.1 -5% (w/w); a preservative, preferably at 0.1 -3% (w/w); an emollient, preferably at 1-3% (w/w); a penetration enhancer, preferably at 1-5% (w/w); a pH adjusting agent, preferably in a quantity sufficient for the composition to maintain a pH of 5-7; and water to make up 100% by weight.

The pharmaceutical product of the invention preferably comprises: palmitoylethanolamide (PEA), or a derivative thereof, the oak roots extract (QR) and the at least one cannabinoid, in the ratios and/or concentrations described above with reference to the composition of the invention.

Therefore, preferably the pharmaceutical product of the invention comprises:
the palmitoylethanolamide (PEA), or a derivative thereof, in a weight ratio with the oak roots extract (QR) of from 1:1 to 1:10, more preferably of from 1:1 to 1:5, most preferably of from 1:1 to 1:3; and/or
the palmitoylethanolamide (PEA), or a derivative thereof, in a weight ratio with the at least one cannabinoid of from 10:1 to 1:10, more preferably of from 5:1 to 1:10, most preferably of from 1:1 to 1: 5; and/or
the at least one cannabinoid in a weight ratio with the oak roots extract (QR) of from 10:1 to 1:10, more preferably of from 5:1 to 1:5, most preferably of from 2:1 to 1:2.

According to preferred embodiments, the pharmaceutical product of the present invention comprises a concentration by weight of PEA, or a derivative thereof, of from 0.01 to 0.2 %, more preferably of from 0.02 to 0.1%, most preferably of from 0.02 to 0.05%, based on the total weight of the pharmaceutical product.

According to further, or alternative, embodiments, the pharmaceutical product of the present invention comprises a concentration by weight of the at least one cannabinoid of from 0.01 to 1%, more preferably from 0.02 to 0.5%, most preferably of from 0.05 to 0.1%, based on the total weight of the pharmaceutical product.

According to further, or alternative, embodiments, the pharmaceutical product of the present invention comprises a concentration by weight of the oak roots extract of from 0.01 to 1%, more preferably from 0.05 to 0.5%, most preferably of from 0.1 to 0.2%, based on the total weight of the pharmaceutical product.

Preferably, the pharmaceutical product of the invention comprises:
a) 0.01-0.5% (w/v) of oak roots extract;
b) 0.01-0.1% (w/v) of palmitoylethanolamide, or a derivative thereof; and
c) 0.01-1% (w/v) of at least one cannabinoid.

According to further preferred embodiments, the pharmaceutical product of the invention comprises:
- a concentration by weight of PEA, or a derivative thereof, of from 0.01 to 0.1%; and/or
- a concentration by weight of the oak roots extract of from 0.02 to 0.1%; and/or
- a concentration by weight of the at least one cannabinoid of from 0.1 to 1%,

based on the total weight of the pharmaceutical product,
and pharmaceutically acceptable excipients.

The total weight of the pharmaceutical product is preferably the sum of the weight of ingredients a), b) and c) and the weight of the pharmaceutically acceptable excipients present in the product.

Preferably, in the pharmaceutical product of the invention, c) the at least one cannabinoid is cannabidiol.

Preferred pharmaceutical products according to the present invention are shown in tables 1-6 that follow, wherein tables 1-3 refer to products in the form of mousse, tables 4-6 refer to a product in the form of a serum:

**Table 1**

| **Dermatological mousse** | |
|---|---|
| **Ingredients** | **w/v %** |
| Sodium Lauroyl Sarcosinate | 1.5 |
| Polyglyceryl-3 Cocoate | 2 |
| Polysorbate 20 | 2 |
| Quercus Robur Root extract | 0.15 |
| Cannabidiol | 0.15 |
| Palmitoylethanolamide | 0.05 |
| Acrylates/C 10-30 Alkyl Acrylate Crosspolymer | 0.2 |
| Water | balance to 100 |

**Table 2**

| **Dermatological mousse** | |
|---|---|
| **Ingredients** | **w/v %** |
| Sodium Lauroyl Sarcosinate | 2 |
| Polysorbate 20 | 2 |
| Polyglyceryl-3 Cocoate | 1.5 |
| Quercus Robur Root extract | 0.2 |
| Cannabidiol | 0.2 |
| Palmitoylethanolamide | 0.1 |
| Acrylates/C10-30 Alkyl Acrylate Crosspolymer | 0.3 |
| Water | balance to 100 |

**Table 3**

| **Dermatological mousse** | |
|---|---|
| **Ingredients** | **w/v %** |
| Sodium Lauroyl Sarcosinate (30% in water) | 1.5 |
| Polysorbate 20 | 1.5 |
| Polyglyceryl-3 Cocoate | 1.5 |
| Quercus Robur Root extract | 0.5 |
| Cannabidiol | 0.3 |
| Palmitoylethanolamide | 0.05 |
| Acrylates/C 10-30 Alkyl Acrylate Crosspolymer | 0.3 |
| Water | balance to 100 |

Optionally, the pharmaceutical products in form of mousse, further comprise one or more of glycine, phenoxyethanol, ethylhexylglycerin, triethanolamine, contributing with water to balance to 100%.

**Table 4**

| **Dermatological serum** | |
|---|---|
| **Ingredients** | **% w/v** |
| Propanediol | 5 |
| Ethanol | 1 |
| Polyglyceryl-3-Laurate | 0.5 |
| Sphingomonas ferment extract | 0.3 |
| Quercus Robur Root extract | 0.08 |
| Cannabidiol | 0.4 |
| Palmitoylethanolamide | 0.05 |
| Water | balance to 100 |

**Table 5**

| **Dermatological serum** | |
|---|---|
| **Ingredients** | **% w/v** |
| Propanediol | 4 |
| Ethanol | 2 |
| Polyglyceryl-3-Laurate | 0.6 |
| Sphingomonas ferment extract | 0.2 |
| Quercus Robur Root extract | 0.1 |
| Cannabidiol | 0.2 |
| Palmitoylethanolamide | 0.04 |
| Water | balance to 100 |

**Table 6**

| **Dermatological serum** | |
|---|---|
| **Ingredients** | **% w/v** |
| Propanediol | 3 |
| Ethanol | 1.5 |
| Polyglyceryl-3-Laurate | 0.7 |
| Sphingomonas ferment extract | 0.5 |
| Quercus Robur Root extract | 0.05 |
| Cannabidiol | 0.5 |
| Palmitoylethanolamide | 0.05 |
| Water | balance to 100 |

Optionally, the pharmaceutical products in form of serum, further comprise one or more of tranexamic acid, Glycerin, Pseudoalteromonas Ferment Extract, Xanthan gum, 4-T-Butylcyclohexanol, Alanine, Proline, Serine, Sodium Phosphate, Pentylene Glycol, Sodium Hydroxide, Tocopherol, Caprylic glycol, Parfum, Ethylhexylglycerin, Sodium Benzoate, Citric acid, contributing with water to balance to 100%.

Preferably, the pharmaceutical product of the invention is prepared by a method comprising the steps of:
- dissolving PEA, or derivative thereof, and the at least one cannabinoid in an aqueous mixture comprising at least one surfactant, preferably comprising polysorbate;
- optionally adding one or more further pharmaceutical excipients to the mixture comprising the at least one cannabinoid;
- adding the oak root extract;
- mixing under stirring the solution, obtaining the product of the invention.

Preferably, said pharmaceutical product is to be administered via external topical, sub-cutaneous topical, mucosal topical, gingival topical, intravesical topical, vaginal topical, rectal topical, or ocular topical route.

In preferred embodiments, said pharmaceutical product is to be administered via external topical route.

Preferably, the pharmaceutical product comprises the composition of the invention in a concentration of 0.1-500 mg/ml of composition, more preferably 1-100 mg/ml.

Said pharmaceutical product may be in the form of ointment, lotion, cream, emulsion, paste, gel, aqueous solution, spray, patch, serum, soaked gauze, dressing, or a combination thereof.

Preferably, the pharmaceutical product further comprises at least one further dermatological active agent, preferably selected from further anti-inflammatory agents, cortisonic agents, antibiotics, antihistaminic agents, anti-acne agents, anti-dandruff agents, anti-psoriasis agents, antifungal agents, antibacterial agents, anti-seborrheic agents, keratolytic agents, humectants, anti-free radical agents, antioxidants, vitamins, and mixtures thereof.

Examples of antifungal agents are econazole and miconazole.

Examples of antibacterial agents are chlorquinol, hexachlorophene, 2,4,4'-trichloro-2'-hydroxybiphenyl ether and usnic acid.

An example of an anti-dandruff agent is ketoconazole.

Examples of anti-isothermal agents are some fatty substances and thiossolone.

Examples of anti-acne agents are retinoic acid and its derivatives.

An example of a keratolytic agent is salicylic acid.

An example of an anti-psoriasis agent is antraline.

All the pharmaceutical compositions described above can be prepared by methods known in the pharmaceutical technique.

In some embodiments, the pharmaceutical product of the invention is for use in the treatment of skin diseases.

The present invention is then directed also to the pharmaceutical product of the invention for use in the treatment of skin hyperpigmentation and of skin inflammatory disease and/or irritant disturbances, optionally associated with hyperpigmentation.

Skin inflammatory diseases preferably include ichthyosis, acne, psoriasis, atopic dermatitis, (eczema), rosacea, skin redness. Said irritant disturbances include skin rashes, urticaria, and itch.

Preferably, invention further relates to the pharmaceutical product of the invention for use in the treatment of skin hyperpigmentation and/or dyschromia.

Preferably the pharmaceutical product of the invention in form of mousse is for use in the treatment of irritant skin disorders.

Preferably the pharmaceutical product of the invention in form of serum is for use in the treatment of hyperpigmentation, dyschromia, and/or of skin inflammation.

It should be understood that all the aspects identified as preferred and advantageous for the composition of the invention are to be deemed as similarly preferred and advantageous also for the pharmaceutical product and uses thereof.

Below are working examples of the present invention provided for illustrative purposes.

### EXAMPLES

### Example 1

Compounds to be tested were prepared by dissolving:
- PEA and cannabidiol in a mixture of surfactants in water;
- QR extract diluted in cell culture medium.

Mixture of the three ingredients were also prepared by mixing the above.

Different concentrations of PEA, cannabidiol and QR extracts, as single ingredients or in admixture, were prepared by serial dilutions.

### Example 2

Cell lines of immortalized melanocytes (B16 cells) were grown at 37 °C, 5% CO₂ in DMEM culture medium, added with 1% L-glutamine and 10% FBS.

The cells were plated in 96-wells multiwell plates at 10⁴ cells/well, in 10% FBS-DMEM. The day after, the culture medium was removed and substituted with 100 µL of complete medium with 1% FBS; then, an MTT assay was carried out after treating cells with the single ingredients prepared in Example 1.

The following concentrations of each ingredient were tested:
1. CBD: 2, 1, 0.5, 0.25, 0.125, 0.063, 0.031, 0.016, 0,008, 0,004 % (w/v)
2. PEA: 0.2, 0.1, 0.05, 0.025, 0.0125, 0.006, 0.003, 0.0015, 0.0007, 0.00035 % (w/v)
3. QR: 1.7, 0.85, 0.425, 0.2125, 0.10625, 0.05321, 0.02652, 0.01326, 0.00663, 0.0034% (w/v).

As positive control of toxicity, sodium dodecyl sulfate (SDS) was used, at the concentration of 1 mg/ml.

The cells were incubated for 24 hours at 37°C and 5% CO₂.

At the end of each incubation, the reagent 3-(4,5-dimethylthiazol-2-yl)-2,5-diphenyltetrazolium bromide (MTT) was added to each well, at the final concentration of 0.45 mg/mL. The plates were then incubated for an additional 2 hours, at 37 °C and 5% CO₂.

At the end of the incubation with MTT, the crystals of reduced MTT were solubilized by removing the medium and adding 100 µL of dimethyl sulfoxide (DMSO) to each well. Finally, the absorbance at 595 nm of each sample was measured, using an Infinite M NANO+ (Tecan) plate reader.

The cell viability following treatment with the different substances was analyzed. Cell viability is expressed as percentage of absorbance at 595 nm of untreated cells (NT).

The test results are shown in Fig. 1.

It is noted that cannabidiol is non-toxic to B16 cells at doses from 0.125% and lower, showing cell vitality equal or greater than 80%.

PEA is generally non-toxic to B16 cells.

QR extract as well is non-toxic to B16 cells, showing cell vitality of 100% even at the highest concentration tested.

### Example 3

B16 cells (immortalized melanocytes) were plated in 48-wells multiwell plates at a density of 6*10⁴ cells/well, in culture medium with 10% FBS. After 24 hours, the culture medium was removed and substituted with 500 µL of complete medium without FBS and, at the same time, hyperproduction of melanin was induced by treatment with A-Melanocyte stimulating hormone (aMSH), as shown in Fig. 2.

Cells were co-treated with each ingredient prepared in Example 1, at the following non-toxic concentrations, as proved in Example 2:
1. CBD: 0.03 %
2. PEA: 0.025%
3. QR: 0.2125 %

Furthermore, treatment with a mixture of the three ingredients were carried out, in order to verify effectiveness and synergy of the composition of the invention In particular, the following mixture was tested, compared to the single ingredients at the same concentration:

**Table 7**

| | CBD (w/v) % | PEA (w/v) % | QR (w/v) % |
|---|---|---|---|
| Mix | 0.03 | 0.025 | 0.2125 |

An experimental group of untreated cells was included, as a negative control.

After 72 hours of treatment, the culture medium was taken away from each well and a wash out was carried out with 500ul/well PBS. Cells were then incubated for 10 minutes with 120 µl of NaOH 1N. Then cells were collected and kept at 70°C for 2 hours in order to dissolve melanin granules. Then 100ul of each sample were transferred in a 96-well plate for spectrophotometric analysis.

Melanin's absorbance was read at 405 nm with an Infinite M Nano+ (Tecan) reader. Melanin production was expressed as % of absorbance of 405 nm vs untreated (NT) cells. As positive control, cells treated with the sole aMSH were included.

Results are shown in Fig. 2 and Table 8 below.

**Table 8**

| | NT | aMSH | CBD | PEA | QR | MIX |
|---|---|---|---|---|---|---|
| Mean % | 100 | 191 | 158 | 122.9 | 179.4 | 97.7 |
| St.dev. | 13.6 | 6.4 | 23.8 | 22.1 | 13.1 | 2.7 |

The mixture showed then a significant and surprising synergic effect, predictive of efficacy of the composition in treating hyperpigmentation.

### Example 4

A pharmaceutical product in form of serum was prepared in accordance with the invention, comprising:
1. CBD: 0.5 % (w/v)
2. PEA: 0.05% (w/v)
3. QR: 0.1 % (w/v)
and suitable pharmaceutical excipients.

A clinical trial was carried out, wherein the activity of said product was evaluated analyzing the reduction of pigmentation and visibility of skin spots (primary endpoints).

The trial also has the following secondary objectives: whether a positive pleasantness and effect of the product are perceived (qualitative discrete secondary endpoint).

The clinical study included 20 enrolled subjects. They were asked to apply the product on face, twice per day for 84 consecutive days. Specific end-point variables were analyzed at baseline time (T0, before the use of the product) and after 28, 56 and 84 days of treatment (respectively: T28, T56 and T84).

The subjects participating in the study were screened under medical supervision and enrolled according to the following inclusion criteria:
- female sex;
- age between 30 and 65 years;
- with all phototypes and with skin hyperpigmentation (melasma - chloasma);
- good general health status/absence of psychological and/or cognitive disorders;
- absence of dermatological and allergological pathologies (cosmetological or to other specific excipients) or other pathologies (such as irritant reactions of unknown origin);
- absence of ongoing pharmacological treatments which may affect the outcome of the test;
- non-participation in other clinical trials in the previous 30 days;
- informed consent shall be obtained.

The subjects received the product without packaging or indications regarding the manufacturer's brand in order to avoid the distortions caused by the conditioning effect of the awareness of the product.

Pigmentation of dark spots (instrumental analysis) was measured by Antera 3D (A.U. arbitrary units). The instrument acquires multiple images and reconstructs the topography of the skin in 3-dimensions, relying on a multi-spectral LED system which illuminates skin surface from different directions (see Fig. 4 as an example). The spectral analysis of the acquired image data allows, through the specific associated software, the study of the concentration of chromophores.

Since the absorption spectrum of melanin is a known value, the software can estimate its concentration. The analysis of the pigmentation of a dark spot represents the average concentration of melanin in the skin.

Visibility of skin spots (clinical evaluation) was evaluated by the professionals responsible for the trial according to the following ordinal scale: Very marked - Marked - Moderate - Slight - Absent.

The rating was given after a visual and/or palpatory clinical study which can involve the use of some suitable equipment for analysis.

Furthermore, when appropriate at the follow up time points the images taken in the areas of interest by specific instruments at the different observation times were viewed and compared.

All measurements and evaluations were carried out following a rest period of at least 20 minutes in an airconditioned room with controlled and regulated temperature and relative humidity (temperature = 21°C +/- 2°C and humidity 40%-60%).

Subjective assessments were carried out asking the subjects enrolled in the trial to fill in a survey, which consists of numerical rating scale questions.

Specifically, the 11-point Numerical Rating Scale (NRS) was used, which ranges from 0 to 10 (0 is the minimum value and 10 the maximum one). The answerers are asked to indicate the numeric value which best describe their response.

The data on the quantitative endpoint were described using the normal position and dispersion measurements: mean and standard deviation/median and interquartile range. A Shapiro-Wilk test was used to verify the normality of the quantitative endpoint variable.

When the assumptions were fulfilled, a parametric one-way repeated measures analysis of variance model was applied in order to evaluate the effect of the treatment over time. When the sphericity assumption was violated, the Greenhouse-Geisser correction was applied. When the result was significant, post-hoc tests were performed were used to compare the observation times of interest: Student's t test with Bonferroni corrections.

When the assumptions were violated, a non-parametric approach was applied. A non-parametric repeated measures analysis of variance model (Friedman test) was used. When the result was significant, post-hoc tests were performed to compare the observation times of interest: after having verified the symmetry of the distribution of the differences between the paired evaluations, the most appropriate non-parametric test for paired data (Wilcoxon signed rank test/Sign test with Bonferroni corrections) was applied.

A significance level of <0.05 was considered.

The data on the qualitative endpoint were described using the normal position and dispersion measurements: mean and standard deviation-median and interquartile range. Furthermore, the absolute frequencies of the number of feedbacks given at each observation period were summarized.

A non-parametric repeated measures analysis of variance model (Friedman test) was applied in order to evaluate the effect of the treatment over time.

When the result was significant, post-hoc tests were performed.

In particular, after having verified the symmetry of the distribution of the differences between the paired evaluations, the most appropriate non-parametric one-tailed or two-tailed test for paired data (Wilcoxon signed rank test/Sign test with Bonferroni corrections) was applied to compare the observation times of interest.

A significance level of <0.05 was considered.

The data on the quantitative discrete endpoints were described using the median. For each self-evaluation question, the percentage frequencies of each score were calculated. Finally, the percentage frequencies of the responses were summarized: responses ≥7 and ≥6 were considered respectively as fully positive and positive.

The conclusions about the self-assessment test were drawn from an overall analysis of the medians of responses to all the questions, as shown in the table 9 below

**Table 9**

| ***Overall median*** | ***Conclusion*** |
|---|---|
| *x < 6* | *Insufficient pleasantness and perceived effect* |
| *6 ≤ x < 7* | *Sufficient pleasantness and perceived effect* |
| *7 ≤ x < 8* | *Moderate pleasantness and perceived effect* |
| *8 ≤ x < 9* | *Good pleasantness and perceived effect* |
| *x ≥ 9* | *Excellent pleasantness and perceived effect* |

During the trial, no subject developed undesirable effects or breached the established inclusion/exclusion criteria. Furthermore, there were no cases of drop-out. Therefore, the analysis refers to a sample of 20 subjects.

### Dark spots pigmentation

Fig. 3 shows the trend of the variable dark spots' pigmentation at the follow-up time points. Data are reported in table 10 below.

**Table 10**

| *Survey Times* | *Mean +st. dev.* | *Median* | IQR |
|---|---|---|---|
| T0 | 52.0 ± 7.2 | 50.6 | 47.2 - 55.7 |
| T28 | 51.1 ± 6.9 | 50.3 | 46.3 - 55.2 |
| T56 | 49.6 ± 7.0 | 49.5 | 44.5 - 51.7 |
| T84 | 48.5 ± 7.3 | 48.4 | 42.3 - 51.5 |

Compared to the baseline value (T0), a decrease of the variable dark spots' pigmentation was observed of:
- 2% after 28 days of treatment;
- 5% after 54 days of treatment;
- 7% after 84 days of treatment.

One-way repeated measures ANOVA test and Post-hoc tests used for pairwise comparisons confirmed a statistically significant effect of the treatment of the invention on the variable dark spots' pigmentation (at least one mean of the variable dark spots pigmentation acquired at all time points is different from the others).

Table 11 shows the absolute frequencies of the judgments at all time points.

**Table 11**

| **Absolute frequency (n. subjects)** | | | | |
|---|---|---|---|---|
| **Judgement** | T0 | T28 | T56 | T84 |
| Very marked | 5 | 4 | 4 | 3 |
| Marked | 10 | 9 | 5 | 6 |
| Moderate | 5 | 6 | 10 | 9 |
| Slight | 0 | 1 | 1 | 2 |
| Absent | 0 | 0 | 0 | 0 |

It is clear that the frequencies of the judgments on the variable visibility of skin spots tend to have a distribution shifting over time to more positive categories.

Specifically, compared to the baseline value (T0), the variable visibility of skin spots improves in the:
- 20% of volunteers after 28 days of treatment;
- 40% of volunteers after 54 days of treatment;
- 50% of volunteers after 84 days of treatment.

Friedman test and confirmed a statistically significant effect of the treatment on the variable visibility of skin spots (at least one of the comparison groups, which represent the variable visibility of skin spots at all time points, is different from the others).

Pairwise post-hoc Sign test (corrected α=0,008) showed statistically significant differences between the baseline value visibility of dark spots and the variable visibility of dark spots after 56 and 84 days of treatment.

65-100% of enrolled subjects gave a positive reply to the questions and the 55-100% a fully positive one, after 84days of treatment.

Furthermore, the medians show overall that the subjects perceived moderate pleasantness and effect of the product, after 84 days of treatment.

### Example 5

Cell lines of immortalized human keratinocyes (HaCaT cells) were grown at 37 °C, 5% CO₂ in DMEM culture medium, added with 1% non-essential amminoacids and 10% FBS. The cells were plated in 96-wells multiwell plates at 10⁴ cells/well, in 10% FBS -DMEM. The day after, the culture medium was removed and substituted with 100 µL of complete medium with 1% FBS; then, an MTT assay was carried out after treating cells with the single ingredients prepared in Example 1.

The following concentrations of each ingredient were tested:
1. CBD: 2, 1, 0.5, 0.25, 0.125, 0.063, 0.031, 0.016, 0,008, 0,004 % (w/v)
2. PEA: 0.2, 0.1, 0.05, 0.025, 0.0125, 0.006, 0.003, 0.0015, 0.0007, 0.00035 % (w/v)
3. QR: 1.7, 0.85, 0.425, 0.2125, 0.10625, 0.05321, 0.02652, 0.01326, 0.00663, 0.0034% As positive control of toxicity, sodium dodecyl sulfate (SDS) was used, at the concentration of 1 mg/ml.

The cells were incubated for 24 hours at 37°C and 5% CO₂.

At the end of each incubation, the reagent 3-(4,5-dimethylthiazol-2-yl)-2,5-diphenyltetrazolium bromide (MTT) was added to each well, at the final concentration of 0.45 mg/mL. The plates were then incubated for an additional 2 hours, at 37 °C and 5% CO₂.

At the end of the incubation, the crystals of reduced MTT were solubilized by removing the medium and adding 100 µL of dimethyl sulfoxide (DMSO) to each well.

Finally, the absorbance at 595 nm of each sample was measured, using an Infinite M NANO+ (Tecan) plate reader.

The cell viability following treatment with the different substances was analyzed. Cell viability is expressed as percentage of absorbance at 595 nm of untreated cells (NT).

The test results are shown in Fig. 5.

It is noted that cannabidiol is non-toxic to HaCaT cells at doses from 0.063% and lower, showing cell vitality equal or greater than 80%.

PEA is generally non-toxic to HaCaT cells with a cell vitality of about 75% at each dose tested, except for the highest one.

QR extract at doses from 0.2% and lower shows cell vitality equal or greater than 80%.

### Example 6

Cell lines of immortalized human keratinocyes (HaCaT cells) were grown at 37 °C, 5% CO2 in DMEM culture medium, added with 1% non-essential amminoacids and 10% FBS. The cells were plated in 48-wells multiwell plates at 5*10⁴ cells/ml, in 10% FBS DMEM. The day after, the culture medium was removed and substituted with 500 µL of complete medium without FBS and at the same time an inflammatory process was induced by treatment with TNFα 20 ng/ml, maintained over/night. This stimulus induces an increase in production of these cytokines compared to controls not receiving TNFα.

An experimental group of untreated cells was included, as a negative control of inflammation.

The following day, the inflamed cells were treated with the ingredients prepared in Example 1, at the following concentrations, proved non-toxic in Example 5:
1. CBD: 0.03, 0.015, 0.0075 %
2. PEA: 0.1, 0.05, 0.025%
3. QR: 0.2125, 0.10625, 0.05321 %

Furthermore, treatment with mixtures of the three ingredients were carried out, in order to verify effectiveness and synergy of the composition of the invention In particular, the following mixture was tested, compared to the single ingredients at the same concentration (respectively numbered as 1, 2 or 3, according to the concentrations used in the relative mixes 1, 2 or 3):

**Table 12**

| | CBD [w/v] | PEA [w/v] | QR [v/v] |
|---|---|---|---|
| MIX 1 | 0.03 | 0.1 | 0.2125, |
| MIX 2 | 0.015 | 0.05 | 0.10625 |
| MIX 3 | 0.0075 | 0.025 | 0.05321 |

After 24 hours of treatment, the culture medium for each well was taken and stored at - 20°C, for subsequent ELISA analyses.

The dosage of the proinflammatory cytokine IL6 (interleukin 6) and IL8 (interleukin 8) was performed on 100 µL of each of the stored aliquots, following the kit protocol provided by the manufacturer. IL6 and IL8 are mediators of inflammation that are produced and released by tissues that suffer an insult, with consequent damage to be repaired. Consequently, the dosage of these cytokines is a valid indicator of inflammatory processes of various nature.

The anti-inflammatory effect of the compounds and compositions testes is expressed as a percentage of interleukins production, with respect to inflamed cells (TNFα-treated) not subjected to anti-inflammatory treatment.

The results are shown in Tables 13-17 that follow.

**Table 13**

| IL-6 production (% vs TNFα) | NT | TNFα | CBD1 | PEA1 | QR1 | MIX1 |
|---|---|---|---|---|---|---|
| Mean | 1 | 100 | 99 | 94 | 101 | 35 |
| St.dev. % | 0.01 | 0.11 | 0.12 | 0.3 | 0.16 | 0.11 |

**Table 14**

| IL-6 production (% vs TNFα) | NT | TNFα | CBD2 | PEA2 | QR2 | MIX2 |
|---|---|---|---|---|---|---|
| Mean | 1 | 100 | 102 | 87 | 94 | 73 |
| St.dev. % | 0.01 | 0.11 | 0.23 | 0.17 | 0.13 | 0.01 |

**Table 15**

| IL-6 production (% vs TNFα) | NT | TNFα | CBD3 | PEA3 | QR3 | MIX3 |
|---|---|---|---|---|---|---|
| Mean | 1 | 100 | 97 | 94 | 87 | 78 |
| St.dev. % | 0.01 | 0.11 | 0.25 | 0.33 | 0.16 | 0.14 |

**Table 16**

| IL-8 production (% vs TNFα) | NT | TNFα | CBD1 | PEA1 | QR1 | MIX1 |
|---|---|---|---|---|---|---|
| Mean | 5 | 100 | 78 | 89 | 84 | 51 |
| St.dev. % | 0.01 | 0.08 | 0.2 | 0.15 | 0.2 | 0.19 |

**Table 17**

| IL-8 production (% vs TNFα) | NT | TNFα | CBD2 | PEA2 | QR2 | MIX2 |
|---|---|---|---|---|---|---|
| Mean | 5 | 100 | 70 | 81 | 83 | 59 |
| St.dev. % | 0.01 | 0.08 | 0.07 | 0.15 | 0.24 | 0.27 |

Only CBD single treatment led to a significative reduction of cell's production of IL8. However, when the ingredients are mixed together, a significative reduction of cell's production of IL6 and IL8 the inflammatory process is shown, thus demonstrating a synergic anti-inflammatory activity of compositions according to the present invention.

Reduction of inflammation in keratinocytes subject to inflammatory insult, by the composition of the invention, is predictive of efficacy of the composition in treating inflammatory skin diseases.

## Claims

1. A cosmetic and/or dermatological composition comprising:
a) oak roots extract;
b) palmitoylethanolamide, or a derivative thereof; and
c) at least one cannabinoid.

2. The cosmetic and/or dermatological composition of claim 1, wherein c) the at least one cannabinoid is selected from: cannabidiol (CBD), cannabidiolic acid (CBDA), cannabinol (CBN), cannabigerol (CBG), cannabichromene (CBC), cannabicyclol (CBL), cannabivarin (CBV), tetrahydrocannabivarin (THCV), cannabidivarin (CBDV), cannabichromevarin (CBCV), cannabigervarin (CBGV), cannabigerol monomethyl ether (CBGM), cannabielsoin (CBE), cannabicitran (CBT), and combinations thereof.

3. The cosmetic and/or dermatological composition of any one of claims 1-2, wherein c) the at least one cannabinoid is cannabigerol (CBG) or cannabidiol (CBD), or a mixture thereof; preferably, wherein c) the at least one cannabinoid is cannabidiol (CBD).

4. The cosmetic and/or dermatological composition of any one of claims 1-3, wherein:
the palmitoylethanolamide (PEA), or a derivative thereof, and the oak roots extract (QR) are in a weight ratio of from 1:1 to 1:10.

5. The cosmetic and/or dermatological composition of any one of claims 1-4, wherein:
the palmitoylethanolamide (PEA), or a derivative thereof, and the oak roots extract (QR) are in a weight ratio of from 1:1 to 1:5, preferably of from 1:1 to 1:3.

6. The cosmetic and/or dermatological composition of any one of claims 1-5, wherein:
the palmitoylethanolamide (PEA), or a derivative thereof, and the at least one cannabinoid are in a weight ratio of from 10: 1 to 1:10.

7. The cosmetic and/or dermatological composition of any one of claims 1-5, wherein:
the palmitoylethanolamide (PEA), or a derivative thereof, and the at least one cannabinoid are in a weight ratio of from 5:1 to 1:10, preferably of from 1:1 to 1:5.

8. The cosmetic and/or dermatological composition of any one of claims 1-7, wherein the at least one cannabinoid and the oak roots extract (QR) are in a weight ratio of from 10:1 to 1:10.

9. The cosmetic and/or dermatological composition of any one of claims 1-8, wherein the at least one cannabinoid and the oak roots extract (QR) are in a weight ratio of from 5:1 to 1:5.

10. The cosmetic and/or dermatological composition of any one of claims 1-9, wherein the at least one cannabinoid and the oak roots extract (QR) are in a weight ratio of from 2:1 to 1:2.

11. The cosmetic and/or dermatological composition of any one of claims 1-10 comprising:
a concentration by weight of PEA, or a derivative thereof, of from 0.01 to 0.2 % w/v,
preferably of from to 0.01 to 0.1%, more preferably of from 0.02 to 0.05%;
and/or a concentration by weight of the at least one cannabinoid of from 0.01 to 1%,
preferably of from 0.02 to 0.5%, more preferably of from 0.05 to 0.2%;
and/or a concentration by weight of the oak roots extract of from 0.01 to 1%, preferably of from 0.05 to 0.5%, more preferably of from 0.1 to 0.2%;
based on the total weight of the composition.

12. A pharmaceutical product comprising the composition of any one of claims 1-11, and pharmaceutically acceptable excipients.

13. The pharmaceutical product of claims 12, further comprising at least one further dermatological active agent, preferably selected from: cortisonic agents, antibiotics, antihistaminic agents, anti-acne agents, anti-dandruff agents, anti-psoriasis agents, antifungal agents, antibacterial agents, anti-seborrheic agents, keratolytic agents, humectants, anti-free radical agents, antioxidants, vitamins, and mixtures thereof.

14. The cosmetic and/or dermatological composition of any one of claims 1-11 or the pharmaceutical product of any one of claims 12-13, for use in the treatment of skin hyperpigmentation and/or dyschromia, and/or of skin inflammatory disease and/or irritant disturbances, optionally associated with hyperpigmentation and/or dyschromia.
